# EUROPEAN PATENT SPECIFICATION

(11) **EP 4 036 106 B1**
(45) Date of publication and mention of the grant of the patent: **10.06.2026**
(21) Application number: 19946979.2
(22) Date of filing: 30.09.2019
(51) Int. Cl.: C07K 14/35, C12N 15/31

(54) **MNEP MONOMER VARIANT AND APPLICATION THEREOF**
MNEP-MONOMERVARIANTE UND IHRE VERWENDUNG
VARIANT DE MONOMÈRE MNEP ET SON APPLICATION

(30) Priority: 29.09.2019 CN 201910936954
(43) Date of publication of application: 03.08.2022
(73) Proprietor: Qitan Technology Ltd., Beijing, Beijing 100192 (CN)
(72) Inventor: LIU, Shaowei, Beijing 100192 (CN); ZHOU, Ya, Beijing 100192 (CN); CHEN, Chengyao, Beijing 100192 (CN)
(74) Representative: HGF
(86) International application number: PCT/CN2019/109333
(87) International publication number: WO 2021/056599

(56) References cited:
- WO-A1-2016/132123
- CN-A- 103 460 040
- CN-A- 106 103 741
- CN-A- 106 459 159
- CN-A- 106 460 061
- CN-A- 107 207 571
- DATABASE GenBank [online] NCBI; 16 May 2016 (2016-05-16), REN, L.: "Mycobacterium adipatum strain YC-RL4, complete sequence.", XP002810546, retrieved from EBI accession no. CP015596 Database accession no. CP015596.1
- DATABASE UniProt [online] 19 February 2014 (2014-02-19), "SubName: Full=Porin {ECO:0000313|EMBL:AHC27123.1};", XP002810547, retrieved from EBI accession no. UNIPROT:V5XH42 Database accession no. V5XH42
- DATABASE PROTEIN 8 August 2018 (2018-08-08), ANONYMOUS: "porin [Mycolicibacterium neoaurum]", XP055795783, retrieved from FASTA Database accession no. AXK74225
- GUO BING-YUAN; ZENG TAO; WU HAI-CHEN: "Recent advances of DNA sequencing via nanopore-based technologies", SCIENCE BULLETIN, ZHONGGUO KEXUE ZAZHISHE, CN, vol. 60, no. 3, 26 December 2014 (2014-12-26), CN, pages 287 - 295, XP035441436, ISSN: 2095-9273, DOI: 10.1007/s11434-014-0707-6

## Description

### Technical Field

The present invention relates to the technical field of characterization of nucleic acid characteristics, in particular to a Mnep monomer variant, a porin and construct including the Mnep monomer variant, and a method for characterizing a target polynucleotide by using the Mnep monomer variant or the porin.

### Background

Nanopore sequencing is a gene sequencing technology. In nanopore sequencing, single-stranded nucleic acid molecules, as sequencing units, are allowed to pass through a nanopore capable of providing an ion current channel under the drive of an electric field force, a corresponding blockage current will be generated due to a physical space occupying effect when polynucleotides are translocated through the nanopore, and different signals generated are read in real time for further analysis to obtain sequence information of the polynucleotides. Nanopore sequencing has the following advantages: easy library construction without amplification; fast reading speed, and the reading speed of single-stranded molecules can reach tens of thousands of bases per hour; longer reading length, usually up to thousands of bases; and direct measurement of DNA or RNA modified by methylation.

However, a specific blockage current will be generated when every or a series of nucleotides pass through a nanoporin under the action of the electric field force. The current signal recorded at this time corresponds to the sequence of the polynucleotides, but usually 3-4 or more nucleotides control the current level at certain grades, so the accuracy still needs to be improved. At present, the accuracy may be improved by changing the polynucleotide structure, duration at the nanopore and developing new nanopores to control translocation of the polynucleotides.

For example, Patent WO2013057495A3 discloses a novel method for characterizing a target polynucleotide, which uses a pore and a Hel308 helicase or a molecular motor capable of binding to the target polynucleotide at an internal nucleotide. The helicase or molecular motor of the invention can effectively control movement of the target polynucleotide through the pore.

Patent CN102216783B discloses a *Mycobacterium smegmatis* porin (Msp) nanopore and sequencing is performed by using the nanopore, wherein the wild-type Msp is mutated at position 90 or 91 to improve conductivity of an analyte during sequencing and reduce translocation rate of the analyte during sequencing.

Patent CN103460040A discloses a mutant Msp monomer and application thereof in nanopore sequencing. The mutant Msp monomer shows stronger ability to distinguish between different nucleotides in nanopore sequencing.

Ren, L. et al. disclose Porin [Mycolicibacterium adipatum] in DATABASE GenBank[Online] NCBI on 16 May 2016.

However, there is no mention of Mnep monomer variants and application thereof in sequencing in the prior art, and there are few porins available for sequencing in the prior art.

Therefore, the present invention further provides a novel nanoporin, and a Mnep monomer variant is prepared by mutating Mnep monomer wild-type protein unavailable for sequencing, and functions of the Mnep monomer variant in sequencing are confirmed.

### Summary

The present invention proves that a preprared Mnep monomer variant with mutation at a specific site of Mnep mutant protein may be used for nanopore sequencing, but a wild-type Mnep monomer does not have the function. In addition, by applying the porin of the present invention in nanopore sequencing may clearly see differences of various nucleotide current signals and has a high sequencing accuracy.

The "Mnep" of the present invention is derived from *Mycobacterium neoaurum.* Preferably, the "Mnep" is derived from *Mycobacterium neoaurum.*

Specifically, a first aspect provides a Mnep monomer variant, and the Mnep monomer variant includes an amino acid sequence with any one or more amino acid mutations of (1) G92K; (2) D93F; (3) G95L; and (4) A104K, and has at least 95% identity to SEQ ID NO: 1.

In one specific implementation of the present invention, the Mnep monomer variant includes mutations of G92K, D93F, G95L and A104K.

Preferably, the Mnep monomer variant also includes an amino acid sequence with any one or more amino acid mutations at positions 80-91 and/or positions 105-120 of SEQ ID NO: 1.

Further preferably, the Mnep monomer variant also includes an amino acid sequence with any one or more amino acid mutations at positions 1-79 and/or positions 121-191 of SEQ ID NO: 1.

Preferably, the variant also includes one or a combination of two or more of mutation of aspartic (D) at position 125, mutation of glutamic (E) at position 141, mutation of glutamic (E) at position 146, mutation of glutamic (E) at position 110, mutation of glycine (G) at position 76, mutation of glycine (G) at position 78, or mutation of glutamine (Q) at position 133.

Further preferably, the variant includes at least one of the following mutations:
D125 is mutated to: lysine (K), glutamine (Q), cysteine (C) or asparagine (N), or modified lysine (K), glutamine (Q), cysteine (C) or asparagine (N), or non-natural amino acid; or
E141 is mutated to: lysine (K), asparagine (N) or glutamine (Q), or modified lysine (K), asparagine (N) or glutamine (Q), or non-natural amino acid; or
E146 is mutated to: arginine (R), asparagine (N) or glutamine (Q), or modified arginine (R), asparagine (N) or glutamine (Q), or non-natural amino acid; or
E110 is mutated to: phenylalanine (F), valine (V), isoleucine (I), leucine (L), alanine (A) or tyrosine (Y), or modified phenylalanine (F), valine (V), isoleucine (I), leucine (L), alanine (A) or tyrosine (Y), or non-natural amino acid; or
G76 is mutated to: serine (S), threonine (T) or arginine (R), or modified serine (S), threonine (T) or arginine (R), or non-natural amino acid; or
G78 is mutated to: serine (S), threonine (T) or arginine (R), or modified serine (S), threonine (T) or arginine (R), or non-natural amino acid; or
Q133 is mutated to: asparagine (N), serine (S) or threonine (T), or modified asparagine (N), serine (S) or threonine (T), or non-natural amino acid.

In one specific implementation of the present invention, the variant may also include at least one of the following mutations:
D125R, E141R, E146K;
D125R, E141R, E146K, E110D;
D125R, E141R, E146K, E110D, G76K, G78K; and
D125R, E141R, E146K, E110D, G76K, G78K, Q133A.

The Mnep monomer variant of the present invention may also include other mutation types other than those described above as long as the mutation does not affect the differentiation of different polynucleotides when the polynucleotides pass through the porin.

Preferably, the variant may also include mutations introducing cysteine to link molecules for sequencing, such as nucleic acid binding proteins.

The Mnep monomer variant of the present invention may only include a narrow region fragment sequence of a porin-forming domain, and retain pore-forming activity. Excess residues may be removed or other amino acid residues may be increased, with the pore-forming activity retained. The length of the fragment may be at least 12, 20, 40, 50, 100 or 150 amino acids.

Preferably, the mnep Monomer variant may be modified to facilitate identification or purification, for example, by adding aspartic residues (asp tag), streptavidin tag, flag tag or histidine residues (His tag).

Preferably, the Mnep monomer variant may have a display marker, for example, fluorescent molecules, radioisotope ¹²⁵I, radioisotope ³⁵S, polynucleotide, biotin, antigen or antibody.

The Mnep monomer variant of the present invention also includes a molecular motor. Preferably, the molecular motor is an enzyme. Further preferably, the enzyme is a polymerase, an exonuclease or a Klenow fragment.

Preferably, the nucleotide sequence encoding the Mnep monomer variant is shown in SEQ ID NO: 11.

A second aspect of the present invention provides a porin including at least one Mnep monomer variant, the Mnep monomer variant includes an amino acid sequence withamino acid mutations of (1) G92K; (2) D93F; (3) G95L; and (4) A104K, and has at least 95% identity to SEQ ID NO: 1, and the mutation results in differences in resistance in a pore due to differences in physical or chemical properties of different types of nucleotides when a single polynucleotide strand passes through the porin including at least one Mnep monomer variant.

Preferably, the mutation results in a change in charge property or hydrophobic property of amino acid.

Preferably, the differences in resistance refer to characteristics that may be used to characterize a polynucleotide, and the characteristics include the source, length, size, molecular weight, identity, sequence, secondary structure and concentration of the polynucleotide or whether a target polynucleotide is modified. Further preferably, the differences in resistance refer to sequence characteristics that may be used to characterize the polynucleotide, that is, the porin may be used for sequencing to accurately distinguish different bases of the polynucleotide.

Preferably, the polynucleotide may be natural or artificially synthesized. Further preferably, the polynucleotide may be natural DNA, RNA or modified DNA or RNA.

Still further preferably, one or more nucleotides in the target polynucleotide may be modified, for example, methylated, oxidized, damaged, abasic, protein-labeled, with tagging or a spacer linked in the middle of the polynucleotide sequence.

Still further preferably, the artificially synthesized nucleic acid is selected from peptide nucleic acid (PNA), glycerol nucleic acid (GNA), threose nucleic acid (TNA), locked nucleic acid (LNA) or other synthetic polymers with nucleoside side chains.

Preferably, the target polynucleotide is single-stranded, double-stranded or at least partially double-stranded.

In one specific implementation of the present invention, the Mnep monomer variant includes mutations of G92K, D93F, G95L and A104K.

Preferably, the Mnep monomer variant also includes an amino acid sequence with any one or more amino acid mutations at positions 80-91 and/or positions 105-120 of SEQ ID NO: 1.

Further preferably, the Mnep monomer variant also includes an amino acid sequence with any one or more amino acid mutations at positions 1-79 and/or positions 121-191 of SEQ ID NO: 1.

Preferably, the Mnep monomer variant also includes one or a combination of two or more of mutation of aspartic (D) at position 125, mutation of glutamic (E) at position 141, mutation of glutamic (E) at position 146, mutation of glutamic (E) at position 110, mutation of glycine (G) at position 76, mutation of glycine (G) at position 78, or mutation of glutamine (Q) at position 133.

Further preferably, the Mnep monomer variant includes at least one of the following mutations:
D125 is mutated to: lysine (K), glutamine (Q), cysteine (C) or asparagine (N), or modified lysine (K), glutamine (Q), cysteine (C) or asparagine (N), or non-natural amino acid; or
E141 is mutated to: lysine (K), asparagine (N) or glutamine (Q), or modified lysine (K), asparagine (N) or glutamine (Q), or non-natural amino acid; or
E146 is mutated to: arginine (R), asparagine (N) or glutamine (Q), or modified arginine (R), asparagine (N) or glutamine (Q), or non-natural amino acid; or
E110 is mutated to: phenylalanine (F), valine (V), isoleucine (I), leucine (L), alanine (A) or tyrosine (Y), or modified phenylalanine (F), valine (V), isoleucine (I), leucine (L), alanine (A) or tyrosine (Y), or non-natural amino acid; or
G76 is mutated to: serine (S), threonine (T) or arginine (R), or modified serine (S), threonine (T) or arginine (R), or non-natural amino acid; or
G78 is mutated to: serine (S), threonine (T) or arginine (R), or modified serine (S), threonine (T) or arginine (R), or non-natural amino acid; or
Q133 is mutated to: asparagine (N), serine (S) or threonine (T), or modified asparagine (N), serine (S) or threonine (T), or non-natural amino acid.

Preferably, the porin includes 1-20 Mnep monomer variants, specifically 1, 2, 3, 4, 5, 6, 7, 8, 9, 10, 11, 12, 13, 14, 15, 16, 17, 18, 19 or 20 Mnep monomer variants, wherein the Mnep monomer variants are identical or different.

Preferably, the porin also includes wild-type Mnep monomers.

In one specific implementation of the present invention, the porin includes 4-10 identical or different Mnep monomer variants.

In one specific implementation of the present invention, the porin includes 4, 6, 8 or 10 Mnep monomer variants.

Preferably, the Mnep monomer variant and the Mnep monomer variant, the wild-type Mnep monomer and the wild-type Mnep monomer, as well as the Mnep monomer variant and the wild-type Mnep monomer are covalently attached.

Preferably, the Mnep monomer variant and the Mnep monomer variant, the wild-type Mnep monomer and the wild-type Mnep monomer, as well as the Mnep monomer variant and the wild-type Mnep monomer are genetically fused.

Preferably, the Mnep monomer variants included in the porin are identical or different. For example, the porin may include eight identical or different Mnep monomer variants. Preferably, the porin includes one Mnep monomer variant and seven identical monomers, wherein the Mnep monomer variant is different from the identical monomers. Alternatively, the porin includes two identical or different Mnep monomer variants and six identical monomers, wherein the Mnep monomer variants are different from the identical monomers. Alternatively, the porin includes three identical or different Mnep monomer variants and five identical monomers, wherein the Mnep monomer variants are different from the identical monomers. Alternatively, the porin includes four identical or different Mnep monomer variants and four identical monomers, wherein the Mnep monomer variants are different from the identical monomers. Alternatively, the porin includes five identical or different Mnep monomer variants and three identical monomers, wherein the Mnep monomer variants are different from the identical monomers. Alternatively, the porin includes six identical or different Mnep monomer variants and two identical monomers, wherein the Mnep monomer variants are different from the identical monomers. Alternatively, the porin includes seven identical or different Mnep monomer variants and one monomer, wherein the Mnep monomer variants are different from the one monomer.

Preferably, the porin includes eight identical or different Mnep monomer variants.

Preferably, the porin may be homologous or heterologous.

Preferably, a pore channel diameter of a narrow region of the porin is less than 18.7Å. Further preferably, the pore channel diameter of the narrow region of the porin is less than 17.2Å.

Still further preferably, the pore channel diameter of the narrow region of the porin is less than 16Å, 15Å, 14Å, 13Å, 12Å, 11Å, 10Å, 9Å or 8Å.

In one specific implementation of the present invention, the pore channel diameter of the narrow region of the porin is about 10Å.

Preferably, the porin allows hydrated ions to flow from one side of a membrane to another layer of the membrane under the drive of an applied potential, wherein the membrane is a bilayer membrane, further preferably a lipid bilayer membrane.

A third aspect of the present invention provides a nucleotide sequence encoding the Mnep monomer variant, or the porin of the present invention.

A fourth aspect of the present invention provides a vector including the nucleotide sequence of the present invention.

Preferably, the vector may provide a plasmid, virus or phage vector which has an origin of replication, a optional promoter used to express the nucleotide sequence, and a regulatory signal gene of the optional promoter. The vector may include one or more selective marker genes, such as a tetracycline resistance gene. The promoter and other expression regulatory signals may be selected to be compatible with a host cell for which an expression vector is designed. The promoter is selected from T7, trc, lac, ara or λL promoters.

The Mnep monomer variant of the present invention may be prepared by chemical synthesis or recombination, preferably by recombination.

Preferably, the vector includes a promoter operably linked to the nucleotide sequence encoding the porin including at least one Mnep monomer variant, the Mnep monomer variant or the construct of the present invention.

Further preferably, the promoter is an inducible promoter or constitutive promoter, wherein the inducible promoter is an acetamide-inducible promoter.

Preferably, the nucleotide sequence encoding the porin including at least one Mnep monomer variant includes at lease one nucleotide sequence encoding the Mnep monomer variant.

Further preferably, the nucleotide sequence encoding the porin including at least one Mnep monomer variant also includes at least one nucleotide sequence encoding the wild-type Mnep monomer.

Still further preferably, the nucleotide sequence encoding the Mnep monomer variant and the nucleotide sequence encoding the Mnep monomer variant, the nucleotide sequence encoding the Mnep monomer variant and the nucleotide sequence encoding the wild-type Mnep monomer, or the nucleotide sequence encoding the wild-type Mnep monomer and the nucleotide sequence encoding the wild-type Mnep monomer are linked by sequence encoding amino acid linker.

A fifth aspect of the present invention provides a mutant bacterium expressing the Mnep monomer variant, or the porin of the present invention. The bacterium includes:
(a) deletion of the wild-type Mnep monomer; and (b) any one of vectors of the present invention.

Preferably, the bacterium includes the vector of the promoter operably linked to the nucleotide sequence encoding the Mnep monomer variant, the construct including the Mnep monomer variant, or the porin including the Mnep monomer variant.

Further preferably, the Mnep monomer variant includes a paralog or homolog of the Mnep monomer variant.

Further preferably, the construct including the Mnep monomer variant includes a construct or monomer of the paralog or homolog of the Mnep monomer variant.

Further preferably, the porin including the Mnep monomer variant includes a porin or monomer of the paralog or homolog of the Mnep monomer variant.

Preferably, the bacterium may also include the vector of the promoter operably linked to the nucleotide sequence encoding the wild-type Mnep monomer, the construct including the wild-type Mnep monomer or the porin including the wild-type Mnep monomer.

Further preferably, the wild-type Mnep monomer includes a monomer of the paralog or homolog of the wild-type Mnep monomer.

Further preferably, the construct including the wild-type Mnep monomer is a construct or monomer of the paralog or homolog of the wild-type Mnep monomer.

Further preferably, the porin including the wild-type Mnep monomer is a porin or monomer of the paralog or homolog of the wild-type Mnep monomer.

Preferably, the bacterium is *Mycobacterium neoaurum.* Further preferably, the bacterium is *Mycobacterium neoaurum.*

A sixth aspect of the present invention provides a method for producing the Mnep porin. The method includes transforming any one of the bacteria of the present invention with any one of vectors of the present invention to induce the bacteria to express the Mnep porin.

A seventh aspect of the present invention provides a method for characterizing a target polynucleotide, including:
(a) contacting the target polynucleotide with the porin of the present invention so that a target polynucleotide sequence passes through the porin; and
(b) obtaining one or more characteristics of an interaction between the nucleotide and the porin when the target polynucleotide passes through the porin to characterize the target polynucleotide.

Preferably, the steps (a) and (b) are repeated for one or more times.

Preferably, the target polynucleotide in the step (a) may be bound to a polynucleotide processing enzyme derived from the polynucleotide, thus controlling a translocation rate. Further preferably, the polynucleotide processing enzyme is a polypeptide capable of interacting with the polynucleotide and modifying at least one of the polynucleotide properties. The polynucleotide processing enzyme may or may not have enzyme activity, as long as the enzyme is bound to the polynucleotide and controls the translocation rate of the polynucleotide in the pore. The nucleic acid may be bound to one or more polynucleotide processing enzymes.

Preferably, the polynucleotide processing enzyme is soluble ribozyme. Further preferably, the polynucleotide processing enzyme includes but is not limited to nucleic acid binding protein, helicase, polymerase, reverse transcriptase, translocase, exonuclease, telomerase or topoisomerase.

In one specific implementation of the present invention, the polynucleotide processing enzyme is gyrase.

Preferably, the step (a) also includes a step of contacting the target polynucleotide with one or a combination of two or more of nucleic acid binding protein, helicase, polymerase, reverse transcriptase, translocase, exonuclease, telomerase and/or topoisomerase, which makes the translocation speed ofthe target polynucleotide sequence passes through the porin less than the translocation speed in the absence of the nucleic acid binding protein, helicase, polymerase, reverse transcriptase, translocase, exonuclease, telomerase and/or topoisomerase.

Further preferably, the nucleic acid binding protein includes but is not limited to one or a combination of two or more of modified or wild-type eukaryotic single-stranded binding protein, bacterial single-stranded binding protein, archaeal single-stranded binding protein, viral single-stranded binding protein or double-stranded binding protein. The nucleic acid binding protein includes but is not limited to SSBEco from *Escherichia coli,* SSBBhe from *Bartonella henselae,* SSBCbu from *Coxiella burnetii,* SSBTma from *Thermathoga maritima,* SSBHpy from *Helicobacter pylori,* SSBDra from *Deinococcus radiodurans,* SSBTaq from *Thermus aquaticus,* SSBMsm from *Mycobacterium smegmatis,* SSBSso from *Sulfolobus solfataricus,* SSBSso7D from *Sulfolobus solfataricus,* SSBMHsmt from *Homo sapiens,* SSBMle from *Mycobacterium leprae,* gp32T4 from *Bacteriohage T4,* gp32RB69 from *Bacteriophage RB69* or gp2.5T7 from *Bacteriohage T7.*

Further preferably, the helicase may be any one of Hel308 family helicase and modified Hel308 family helicase, RecD helicase and its variants, TrwC helicase and its variants , Dda helicase and its variants, TraI Eco and its variants, or XPD Mbu and its variants.

Further preferably, the polymerase includes but is not limited to modified or wild-type DNA polymerase, including but not limited to Phi29 DNA polymerase, Tts DNA polymerase, M2DNA polymerase, VENT DNA polymerase, T5DNA polymerase, PRD1DNA polymerase, Bst DNA polymerase or REPLI-gscDNA polymerase.

Further preferably, the exonuclease includes but is not limited to modified or wild-type exonuclease I from *Escherichia coli,* exonuclease III from *Escherichia coli,* exonuclease from phageλor RecJ from *Thermus thermophilus.*

In one specific implementation of the present invention, the step (a) includes a step of contacting the target polynucleotide with the helicase, the helicase is EF8813, the amino acid sequence of the helicase is shown in SEQ ID NO: 3, and the nucleotide sequence of the helicase is shown in SEQ ID NO: 4. Preferably, the target polynucleotide may be contacted with one or more helicases. Further preferably, the target polynucleotide may be contacted with 2-20 helicases or even more helicases. The helicases that binds to the target polynucleotide may be identical or different. The multiple helicases binding to the target polynucleotide are covalently linked to each other.

Preferably, the one or more characteristics are selected from the source, length, size, molecular weight, identity, sequence, secondary structure and concentration of the target polynucleotide or whether the target polynucleotide is modified.

In one specific implementation of the present invention, the characteristic is a sequence.

Preferably, the one or more characteristics in the step (b) are carried out by electrical measurement and/or optical measurement.

Further preferably, electrical signals and/or optical signals are generated by the electrical measurement and/or optical measurements, and each nucleotide corresponds to a signal level, and then the electrical signals and/or optical signals are converted into nucleotide sequence characteristics.

The electrical measurement of the present invention is selected from current measurement, impedance measurement, field effect transistor (FET) measurement, tunnel measurement or wind tunnel measurement.

The electrical signal of the present invention is selected from current, voltage, tunneling, resistance, potential, conductivity or a measured value of transverse electric measurement.

In one specific implementation of the present invention, the electrical signal is the current passing through the pore. That is, the current passes through the pore in a nucleotide-specific manner, and if a nucleotide-related characteristic current is detected to flow through the pore, the nucleotide is present, and vice versa, the nucleotide is not present. However, the distinction between similar nucleotides or modified nucleotides is determined by the magnitude of the current.

Preferably, conductivity generated in the process of characterizing the polynucleotide with the porin of the present invention is higher than that of the pore formed by the wild-type Mnep monomer.

Preferably, the method also includes a step of applying a potential difference across the porin contacted with the target polynucleotide, wherein the potential difference is sufficient to translocate the target polynucleotide from a porin channel.

Preferably, the target polynucleotide may be natural DNA, RNA or modified DNA or RNA.

The target polynucleotide of the present invention is a macromolecule including one or more nucleotides.

The target polynucleotide of the present invention may be natural or artificially synthesized. Preferably, one or more nucleotides in the target polynucleotide may be modified, for example, methylated, oxidized, damaged, abasic, protein-labeled, with tagging nucleotide or a spacer linked in the middle of the polynucleotide sequence. Preferably, the artificially synthesized nucleic acid is selected from peptide nucleic acid (PNA), glycerol nucleic acid (GNA), threose nucleic acid (TNA), locked nucleic acid (LNA) or other synthetic polymers with nucleoside side chains.

Preferably, the porin allows hydrated ions to flow from one side of a membrane to another layer of the membrane under the drive of an applied potential, wherein the membrane may form a barrier for the flow of ions, nucleotides and nucleic acids. Further preferably, the membrane is a bilayer membrane, further preferably a lipid bilayer membrane. The lipid bilayer membrane includes but is not limited to one or a mixture of two or more of phospholipid, glycolipid, cholesterol and mycolic acid.

Preferably, the porin channel is located between a first conductive liquid medium and a second conductive liquid medium, wherein at least one conductive liquid medium includes the target polynucleotide, and the first conductive liquid medium and the second conductive liquid medium may be identical or different, as long as the purpose of analyzing one or more characteristics of the target polynucleotide may be achieved.

Preferably, the target polynucleotide is single-stranded, double-stranded or at least partially double-stranded.

In one specific implementation the target polynucleotide is at least partially double-stranded, wherein the double-stranded part constitutes a Y-adaptor structure, the Y-adaptor structure includes a leader sequence that is preferentially screwed into the porin, and a 3' terminal of the leader sequence is attached to mercaptan, biotin or cholesterol for binding to a layer of the lipid bilayer membraneto point the target polynucleotide in a right direction and give a pulling effect.

In one specific implementation the 3' terminal of the leader sequence is attached to cholesterol for binding to a layer of the lipid bilayer membrane.

The degree of differentiation of different nucleotides in the characterization of the target polynucleotide by the porin of the present invention may be controlled by regulating the voltage, salt concentration, buffer, additive or temperature in the characterization of the target polynucleotide. The additive is selected from DTT, urea or betaine.

Preferably, the voltage ranges is from -250 mV to +250 mV. Further preferably, the voltage is selected from -250 mV, -210 mV, -180 mV, -140 mV, -110 mV, -90 mV, -70 mV, -40 mV, 0 mV, +40 mV, +70 mV, +90 mV, +110 mV, +140 mV, +180 mV, +210 mV or +250 mV.

In one specific implementation of the present invention, the voltage is from -180 mV to +180 mV.

In one specific implementation the method includes inserting the porin into the membrane, and then contacting the target polynucleotide with the porin, nucleic acid binding protein, polymerase, reverse transcriptase, translocase, exonuclease, topoisomerase, telomerase or helicase, and applying a potential difference across the porin contacted with the target polynucleotide so that the target polynucleotide sequence passes through the porin; and
obtaining current characteristics of an interaction between the nucleotide and the porin when the target polynucleotide passes through the porin to identify whether the polynucleotide is present, which nucleotide is the polynucleotide or whether the polynucleotide is modified.

Preferably, the method of inserting the porin into the membrane may be any method known in the art to achieve the purpose of characterizing polynucleotides. Further preferably, the porin may be suspended in a purified form in a solution including a lipid bilayer, so that the porin diffuses into the lipid bilayer and is inserted into the lipid bilayer by binding to the lipid bilayer and assembling into a functional state.

The "non-natural amino acid" of the present invention is compounds which are not found naturally in proteins and include amino and carboxyl groups. Preferably, the non-natural amino acids are any non-natural amino acids known in the art. Further preferably, the non-natural amino acids include but are not limited to N-ethyl aspartyl, hydroxylysine, 3-hydroxyproline, 2-aminobutyric acid, β-alanine, β-amino propionic acid, 2-aminoadipic acid, 3-aminoadipic acid, 4-aminobutyric acid, 6-aminocaproic acid, 2-aminoheptanic acid, allo-isoleucine, isochainlysine, 4-hydroxyproline, allo-hydroxylysine, 2-aminoisobutyric acid, N-methylglycine, N-methy isoleucine, 3-aminoisobutyric acid, 6-N-methyllysine, 2,4-diaminobutyric acid, N-methylvaline, ornithine, norleucine, norvaline, desmosine, 2,2'-diaminopimelic acid, 2,3-diaminopropionic acid, N-ethyl glycine or 2-amino heptanedioic, etc.

The "modified...amino acid" of the present invention is an amino acid which the side chain is chemically modified, for example, a post-translationally modified amino acid, or an amino acid with a side chain including a novel functional group (e.g. sulfhydryl, amino or carboxyl) or with a side chain including a part that generates a signal (e.g. a fluorophore or radioactive label).

The "pore channel diameter of the narrow region" of the present invention refers to a diameter of a narrowest portion of a cross section of a pore inside the porin.

The "nucleotide" of the present invention includes but is not limited to adenosine monophosphate (AMP), guanosine monophosphate (GMP), thymidine monophosphate (TMP), uridine monophosphate (UMP), cytosine nucleoside monophosphate (CMP), cyclic adenosine monophosphate (cAMP), cyclic guanosine monophosphate (cGMP), deoxyadenosine monophosphate (dAMP), deoxyguanosine monophosphate (dGMP), deoxythymidine monophosphate (dTMP), deoxyuridine monophosphate (dUMP) and deoxycytidine monophosphate (dCMP). Preferably, the nucleotide is selected from AMP, TMP, GMP, CMP, UMP, dAMP, dTMP, dGMP or dCMP.

The "and/or" of the present invention includes an alternatively listed items and a combination of any number of items.

The "include/including", "contain" and "comprise" of the present invention is an open description, and contains the specified components or steps described, and other specified components or steps that will not have an affect substantially.

The "about" of the present invention is used to represent the value and the standard deviation allowed by the apparatus or method for measuring the value.

The "homology", "homolog" and "homologous" of the present invention refers to that in terms of use of a protein sequence or nucleotide sequence, those of ordinary skilled in the art may adjust the sequence according to the actual work needs, so that compared with the sequence obtained by the prior art, the sequence used has (including but not limited to) 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 70%, 80%, 81%, 82%, 83%, 84%, 85%, 86%, 87%, 88%, 89%, 90%, 91%, 92%, 93%, 94%, 95%, 96%, 97%, 98%, 99%, 99.1%, 99.2%, 99.3%, 99.4%, 99.5%, 99.6%, 99.7%, 99.8% or 99.9% sequence identity.

The "Mnep monomer variant" of the present invention refers to a Mnep monomer variant that has at least or at most 70%, 75%, 80%, 85%, 90%, 91%, 92%, 93%, 94%, 95%, 96%, 97%, 98%, 99%, 99.5% or 99.9% or more, or any range there between, but less than 100% identity with the wild-type Mnep monomer, and retains a channel-forming ability when binding to one or more other Mnep monomer variants or wild-type Mnep monomers. Optionally, the Mnep monomer variant is further identified to include mutations in the portion of the sequence that promotes the formation of the narrow region of the fully formed channel-forming porin. The Mnep monomer variant may be, for example, a recombinant protein. The Mnep monomer variant may include any mutations described herein.

The "porin of the paralog or homolog of the Mnep monomer variant" of the present invention refers to a porin of the paralog or homolog of the Mnep monomer variant that has at least or at most 70%, 75%, 80%, 85%, 90%, 91%, 92%, 93%, 94%, 95%, 96%, 97%, 98%, 99%, 99.5% or 99.9% or more, or any range there between, but less than 100% identity with the porin of the paralog or homolog of the wild-type Mnep monomer, and retains the channel-forming ability. Optionally, the porin of the paralog or homolog of the Mnep monomer variant is further identified to include mutations in the portion of the sequence that promotes the formation of the narrow region of the fully formed channel-forming porin. The porin of the paralog or homolog of the Mnep monomer variant may be, for example, a recombinant protein. The porin of the paralog or homolog of any Mnep monomer variant may be optionally used for any implementation herein.

### Brief Description of the Drawings

Embodiments of the present invention are described in detail below with reference to the attached drawings, wherein:

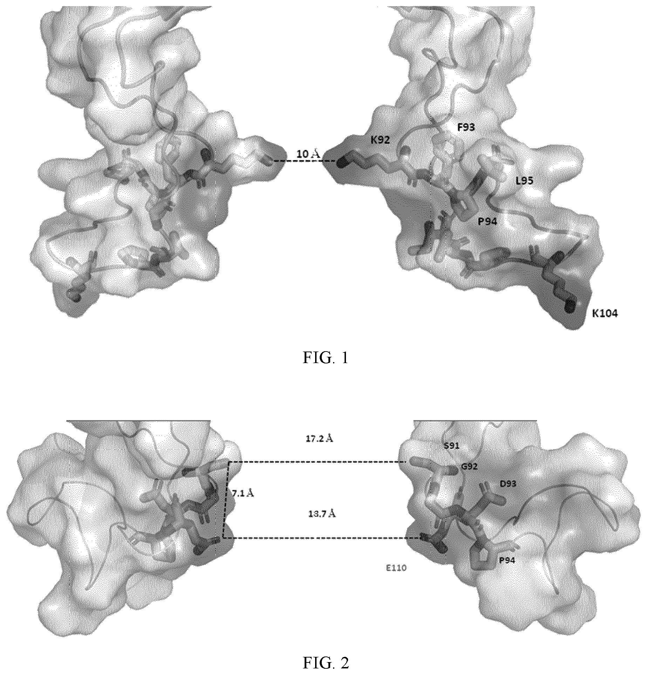

FIG. 1: a stick model of a nanopore (G92K/D93F/G95L/A104K) comprising a Mnep monomer variant, showing distribution characteristics of amino acids in a narrow region of the pore channel, with homology modeling completed by SWISS MODEL and a template pdb is 1uun.
FIG. 2: a stick model of a wild-type Mnep nanopore, showing distribution characteristics of amino acids in a narrow region of the pore channel, with homology modeling completed by SWISS MODEL and a template pdb is 1uun.

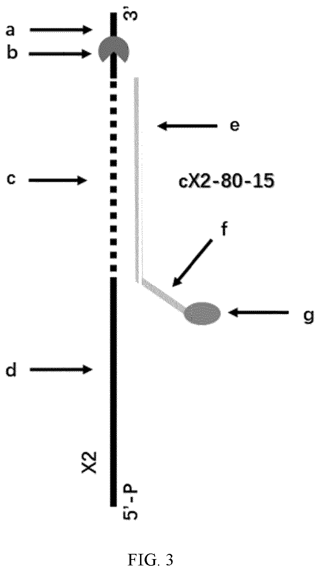

FIG. 3: a structure diagram of a DNA construct X2& cX2-80-15 to be tested, in which a segment a corresponds to SEQ ID NO: 7, a segment b corresponds to helicase EF8813-1 (including a N terminal histidine tag and a variant protein of fused with a TOPV-HI domain, SEQ ID NO: 3-4), the helicase may bind to the segment tagged as a, a segment c corresponds to SEQ ID NO: 6, a segment d corresponds to SEQ ID NO: 5, a segment e corresponds to SEQ ID NO: 8, 45 bases at the 5' terminal of cX2-80-15 are complementary to the segment c of a test chain for pairing, and a 3' terminal of cX2-80-15 contains 40 thymines and 3' cholesterol TEG marker corresponding to a segment g, and a segment f corresponds to SEQ ID NO: 9.

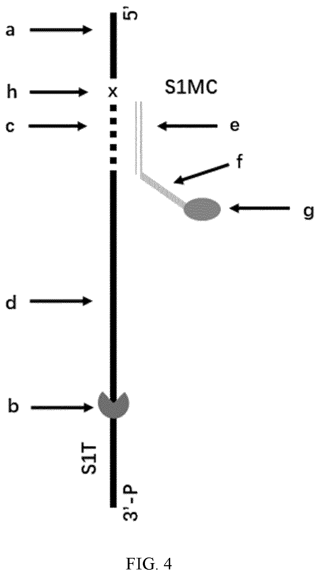

FIG. 4: a structure diagram of a DNA construct S1T&S1MC to be tested, in which a segment a corresponds to SEQ ID NO: 10, a segment b corresponds to helicase EF8813-1 (including a N terminal histidine tag and a variant protein of fused with a TOPV-HI domain, SEQ ID NO: 3-4), the helicase may bind to the segment tagged as a, a segment h refers to dspacer that only retains the phosphoric acid skeleton and is labeled as x, a segment c corresponds to SEQ ID NO: 12, a segment d corresponds to SEQ ID NO: 13, a segment e corresponds to SEQ ID NO: 14, 45 bases at a 5' terminal of S1MC are complementary to the segment c of a test chain for pairing, and a 3' terminal of S1MC contains 20 thymines and a 3' cholesterol TEG marker corresponding to a segment g, and a segment f corresponds to SEQ ID NO: 15.

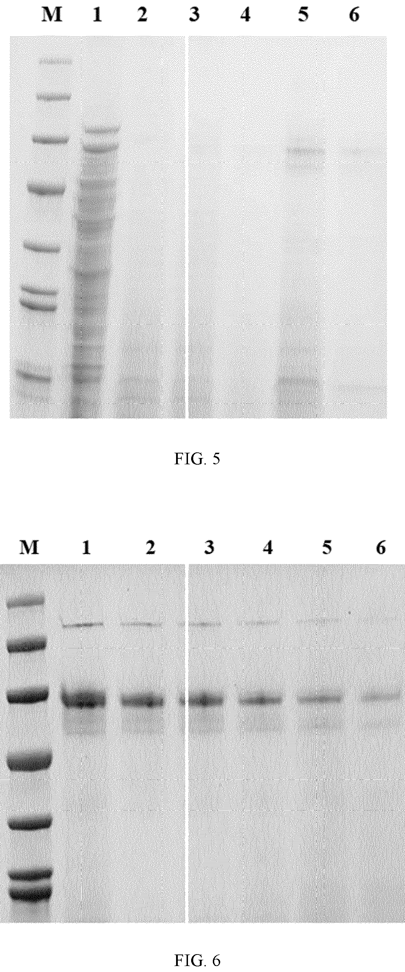

FIG. 5: a purification result of Mnep-(G92K/D93F/G95L/A104K) monomer variant protein by anion exchange column chromatography, in which a first lane is a broken whole cell lysate, a second lane is a supernatant of the whole cell lysate after centrifugation, a third lane is a penetrating component of the anion exchange column, and fourth, fifth and sixth lanes are three eluting peaks of NaCl linear elution, and the results show that the second eluting peak (a result shown in a fifth lane) contains the highest amount of target protein.
FIG. 6: a purification result of Mnep-(G92K/D93F/G95L/A104K) monomer variant protein by molecular sieve exclusion chromatography, in which the first to sixth lanes show the electrophoresis results of different components collected from the molecular sieve.

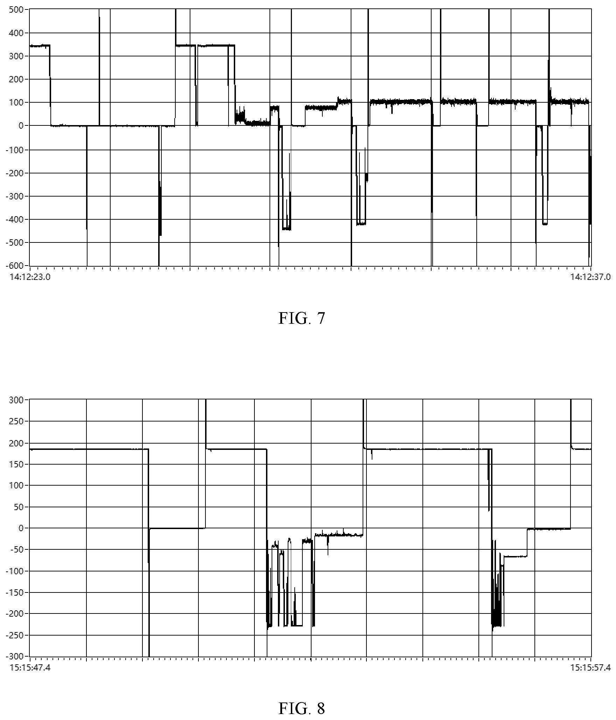

FIG. 7: single channel behavior characteristics of a wild-type Mnep monomer pore channel at ±180 mV, wherein y-axis coordinate = current (pA) and x-axis coordinate = time (s).
FIG. 8: open-pore current and gating characteristics of a Mnep-(G92K/D93F/G95L/A104K) monomer variant at +180 mV, 0 mV and -180 mV, wherein y-axis coordinate = current (pA) and x-axis coordinate = time (s).

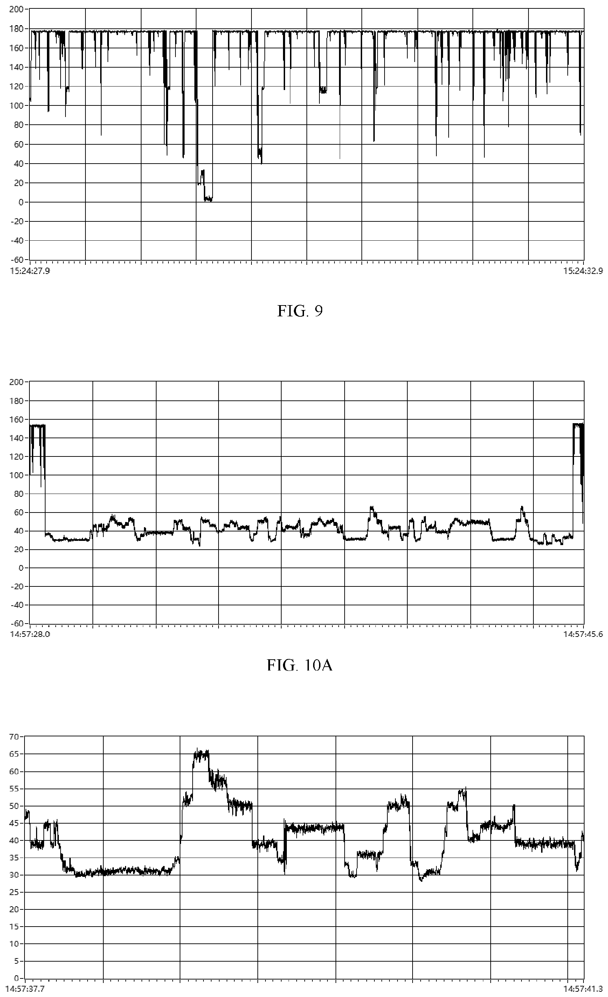

FIG. 9: a signal of nucleic acid passing through a nanopore (Mnep-(G92K/D93F/G95L/A104K) including a Mnep monomer variant at +180 mV, wherein y-axis coordinate = current (pA) and x-axis coordinate = time (s).

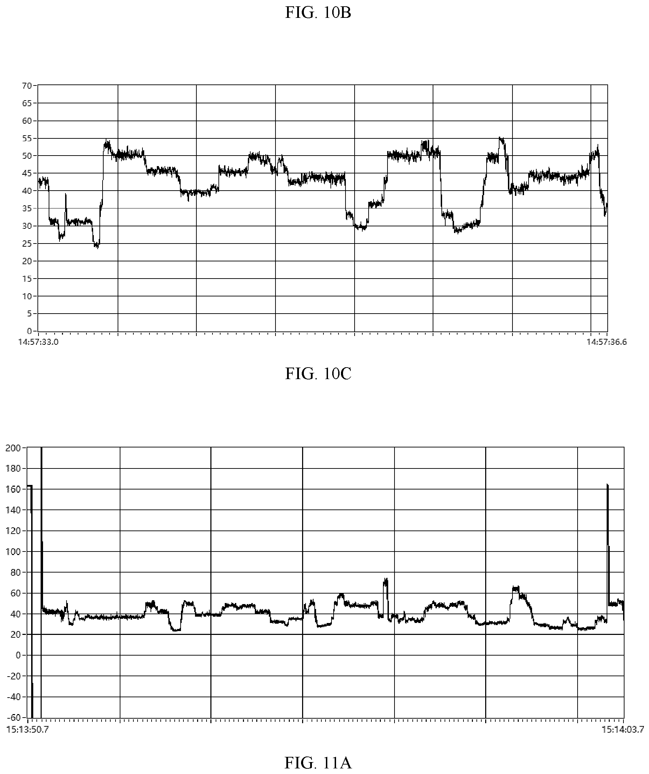

FIG. 10A: example current trajectories when helicase (EF8813-1) controls a DNA construct X2&cX2-80-15 to translocate through a nanopore including a Mnep-(G92K/D93F/G95L/A104K) monomer variant, wherein y-axis coordinate of two trajectories = current (pA) and x-axis coordinate = time (s); and FIG. 10B and FIG. 10C show amplification results of the current trajectories in some areas of FIG. 10A.

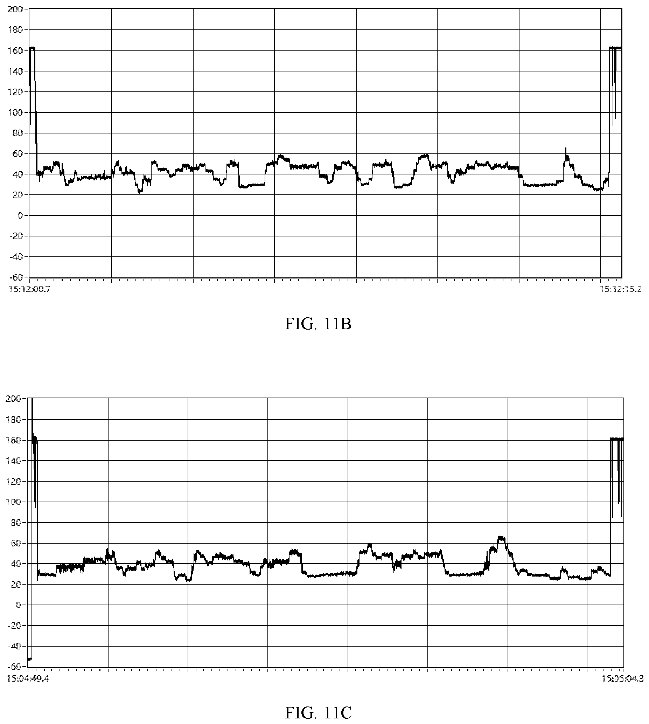

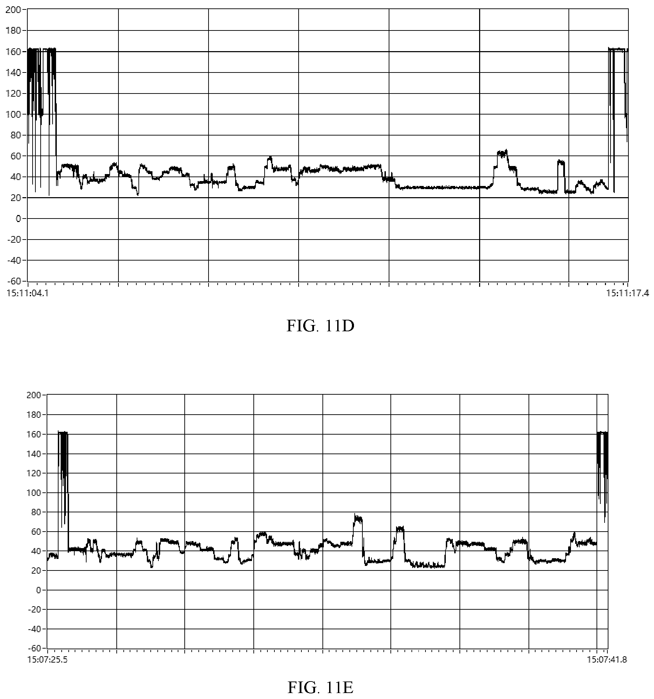

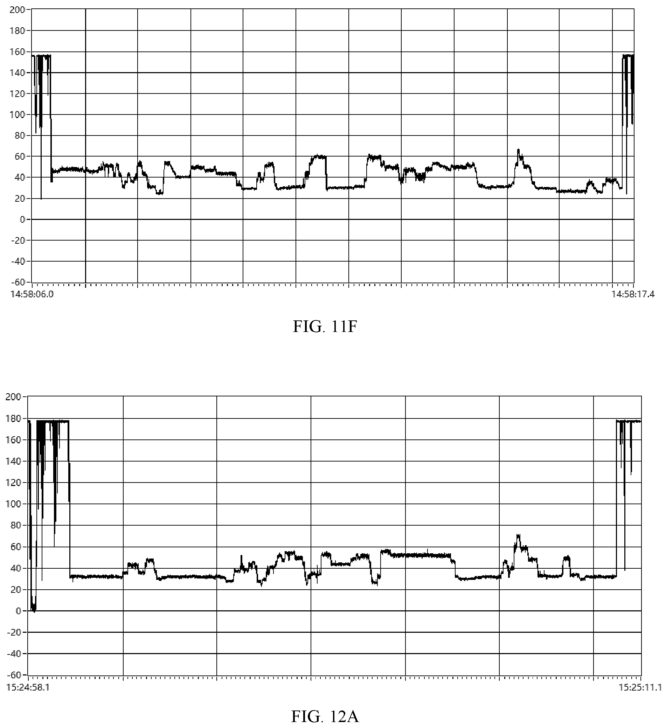

FIG. 11: FIG. A, B, C, D, E and F are the results of different segments of the example current trajectories respectively when the helicase (EF8813-1) controls the DNA construct X2&cX2-80-15 to translocate through the nanopore including the Mnep-(G92K/D93F/G95L/A104K) monomer variant, wherein y-axis coordinates of the two trajectories = current (pA) and x-axis coordinates = time (s).

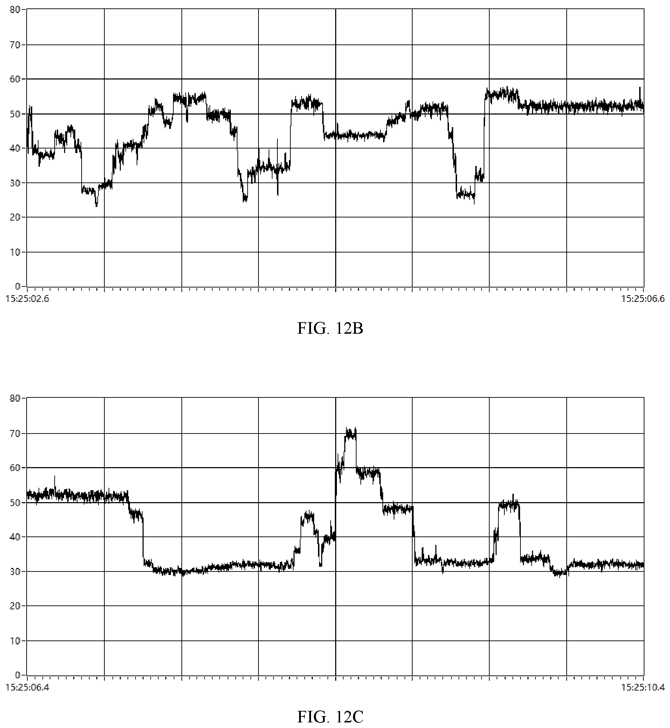

FIG. 12A: an example current trajectory when helicase (EF8813-1) controls a DNA construct S1T&S1MC to translocate through a nanopore including a Mnep-(G92K/D93F/G95L/A104K) monomer variant. FIG. 12B and 12C are the amplification results of the current trajectory in some areas of FIG. 12A. The maximum current value indicated by an arrow in FIG. 12C shows a characteristic peak of dspacer, wherein y-axis coordinates of the two trajectories = current (pA), and x-axis coordinates = time (s).

### Detailed Description of the Embodiments

Technical solutions in the embodiments of the present invention are described clearly and completely below with reference to the attached drawings in the embodiments of the present invention.

### Embodiment 1 Preparation of Mnep monomer variant

### I. Construction of plasmid

A protein sequence of a Mnep monomer variant was optimized by codons of corresponding amino acids, and suitable restriction endonuclease sites were added at both ends of a gene. Specifically, a NcoI site ccatgg was added at a 5' terminal, and an xhoI site ctcgag was added at a 3' terminal. Then the gene was synthesized, and the synthesized gene was cloned into an expression vector pET24b.

### II. Preparing a nucleotide sequence of the Mnep monomer variant by site-directed mutagenesis of a target gene

A mutant gene was induced (a PCR reaction) to perform PCR amplification with designed primers and KOD plus high-fidelity enzyme by using a plasmid to be mutated as a template, thus inducing mutation of the target gene.

The specific steps are as follows:
1. Point mutation primers were designed, and template plasmid DNA (including plasmid DNA of SEQ ID NO: 2) was prepared and amplified with a 50µL PCR reaction system. A DH5α strain was used as host bacterium. In an end+ type strain, the clone number is often low, but the mutation efficiency is not affected. Wherein, a QIGEN plasmid purification kit was used to extract the template plasmid DNA.

### Point mutation primers:

SEQ ID NO: 16 
CCACCCCGAATGTGGCACTGAGTAAATTTCCGCTGTTTGGCATTACCGGCATTGGCG
GCAAACTGCCGGTGATTGGCGAAATTG
SEQ ID NO: 17

CAATTTCGCCAATCACCGGCAGTTTGCCGCCAATGCCGGTAATGCCAAACAGCGGA

AATTTACTCAGTGCCACATTCGGGGTGG

### 50µL PCR reaction system:

| | |
|---|---|
| 10×KOD plus Buffer | 5 µL |
| Template plasmid DNA 60ng | 2µL |
| Forward primer (20pmol/µL) | 2µL |
| Reverse primer (20pmol/µL) | 2µL |
| dNTP mixture (each 2.5mM) | 2µL |
| MgSO₄ | 2.5 µL |
| KOD plus enzyme | 1µL |
| ddH₂O | 33.5µL |

### PCR amplification reaction

### Cycles, temperature and reaction time:

| | | |
|---|---|---|
| 1 cycle | 95°C | 5min |
| 18 cycles | 95°C | 30s |
| | 72°C | 30s 6min |
| | 55°C | 30min |
| 1 cycle | 72°C | 10min |

After the PCR amplification reaction is completed, the nucleotide sequence of the Mnep monomer variant (SEQ ID NO: 11) was obtained. A resulting product was kept in an ice bath for 5min, and then kept at room temperature (to avoid repeated freezing and thawing).

### 2. Extracting Mnep monomer variant gene by template digestion

After the PCR reaction is finished, a methylated plasmid was digested with DpnI enzyme to select mutant plasmid DNA (a plasmid including SEQ ID NO: 11). A PCR reaction product was prepared specifically by adding 1µL (10 U/µL) of DpnI enzyme and incubating at 37°C for 2 hours. (When the amount of plasmid DNA is too much, the DpnI enzyme may react incompletely with the sample. If the mutation rate is low, the reaction time may be appropriately prolonged or the amount of the DpnI enzyme may be increased.)

### 3. Obtaining a strain containing the Mnep monomer variant gene by transformation

After the reaction, a gap was formed in the plasmid DNA. Therefore, when the plasmid DNA was transferred into E. coli, DH5α was selected specifically by the following steps: adding 4 µL of a mutant plasmid DNA sample to 50 µL of DH5α competent cells, and then keeping on ice for 30min, thermal shocking at 42°C for 90 seconds, then immediately keeping in an ice bath for 2min, adding 500 µL of an SOC medium, culturing at 37°C for 1 hour, and finally taked 100 µL bacterial solution to coat the a resistance screening plate.

### 4. Sequencing validation

Picked four transformants for culturing and sequencing, and positive transformants with correct mutation were selected to extract plasmids to be stored for standby application.

### III. Preparation of the Mnep monomer variant

The Mnep monomer variant plasmid validated by sequencing correctly was transferred into BL21(DE3) for culture. Then the protein was purified, wherein the formulae of the reagents for protein purification are shown in Table 1.

Pipetted 12µL of Mnep-KO BL21 (DE3) glycerol bacteria into 12mL (1:1000) fresh LB medium with a final concentration of 50mg/mL kanamycin, 37°C and 200 rpm shaked overnight for activation, and amplification culture was performed according to an inoculation amount of 1% to a 2L LB medium with a final concentration of 50mg/mL kanamycin on the next day. After culturing at 37°C and 220 rpm to OD600=0.6-0.8, the resulting solution was kept in an ice bath for rapid cooling, and then isopropyl thiogalactoside (IPTG) with a final concentration of 1mM was added to the culture system, and expression was induced overnight at 18°C and 220 rpm.

The bacteria were collected by centrifugation at 6000 rpm and 4°C for 15min on the next day. The bacteria were resuspended at a ratio of bacteria: lysis buffer=1:10 (m/v), then a mixed protease inhibitor was added, and high pressure crushed was carried out until the bacterial solution became clear.

1% OPOE (octylphenol polyoxyethylene ether) and 0.1% FC12 (N-dodecylphosphorylcholine) were added and stirred at room temperature for solubilization for 1-2 hours. The solubilized lysis products were treated in a boiling water bath for 20min, and then kept in an ice bath for 60min immediately. After that, PEI (polyetherimide) with a final concentration of 0.3% was added. After thorough mixing, the resulting solution was kept on ice for 5min, and centrifuged at 4°C and 14000 rpm for 30min, and the supernatant was collected.

The supernatant was filtered by a 0.45µm filter membrane and purified by an anion exchange column. The ion column was pre-equilibrated with a Buffer B. The supernatant flowed through the column at a flow rate of 5mL/min, and then miscellaneous proteins were eluted with the Buffer B. At last, buffer C: 0-1M salt concentration was used for linear gradient elution, and the eluted components were collected. The results of purification by the anion exchange column are shown in FIG. 5.

The eluted sample collected was concentrated in a 100kDa ultrafiltration tube, and centrifuged at 4°C and 14000 rpm for 20min, and the supernatant was retained; a proper amount of the concentrated supernatant was subjected to molecular exclusion chromatography. The chromatographic column were equilibrated with SEC Buffer of the two column volumes in advance. The sample was loaded, concentrated and detected by SDS-PAGE gel electrophoresis. The results of molecular exclusion chromatography are shown in FIG. 6.

**Table 1 Formulae of reagents for protein purification**

| Reagent name | Component |
|---|---|
| Lysis buffer | 50mM Tris-HCl 8.0 |
| | 150mM NaCl |
| | 10% glycerol |
| Buffer B | 25mM HEPES-Na 7.5 |
| | 0.5% OPOE |
| Buffer C | 25mM HEPES-Na 7.5 |
| | 1M NaCl |
| | 0.5% OPOE |
| SEC buffer | 25mM HEPES-Na 7.5 |
| | 150mM NaCl |
| | 0.5% OPOE |

### Embodiment 2 Preparation of porin

1. Pipetted 12µL of Mnep-KO BL21 (DE3) glycerol bacteria into 12mL (1:1000) fresh LB medium with a final concentration of 50mg/mL kanamycin, 37°C and 200 rpm shaked overnight for activation, and amplification culture was performed according to an inoculation amount of 1% to a 2L LB medium with a final concentration of 50mg/mL kanamycin on the next day. After culturing at 37°C and 220 rpm to OD₆₀₀=0.6-0.8, the resulting solution was kept in an ice bath for rapid cooling, and then IPTG with a final concentration of 1mM was added to the culture system, and expression was induced overnight at 18°C and 220 rpm.
2. The bacteria were collected by centrifugation at 6000 rpm and 4°C for 15min on the next day. The bacteria were resuspended at a ratio of bacteria: lysis buffer=1:10 (m/v), then a mixed protease inhibitor was added, and high pressure crushedwas carried out until the bacterial solution became clear.
3. 1% OPOE and 0.1% FC12 were added and stirred at room temperature for solubilization for 1-2 hours.
4. The solubilized lysis products were treated in a boiling water bath for 20min, and then kept in an ice bath for 60min immediately. After that, PEI with a final concentration of 0.3% was added. After thorough mixing, the resulting solution was kept on ice for 5min, and centrifuged at 4°C and 14000 rpm for 30min, and the supernatant was collected.
5. The supernatant was filtered by a 0.45µm filter membrane and purified by an anion exchange column. The ion column was pre-equilibrated with a Buffer B. The supernatant flowed through the column at a flow rate of 5mL/min, and then miscellaneous proteins were eluted with a Buffer B. At last, buffer C: 0-1M salt concentration was used for linear gradient elution, and the eluted components were collected.
6. The eluted sample collected was concentrated in a 100kDa ultrafiltration tube, and centrifuged at 4°C and 14000 rpm for 20min, and the supernatant was retained; a proper amount of the concentrated supernatant was subjected to molecular exclusion chromatography. The chromatographic column were equilibrated with SEC Buffer of the two column volumes in advance. The sample was loaded, concentrated and detected by SDS-PAGE gel electrophoresis.

### Embodiment 3 Application of porin in sequencing

A single nanopore was inserted into a phospholipid bilayer in a buffer (400mM KCl, 10mM HEPES pH 8.0, 50mM MgCl₂), and electrical measurement values were obtained from the single nanopore.

The specific steps are as follows:
After inserting a single porin (a Mnep-KO nanopore, a stick model shown in FIG. 1) of SEQ ID NO:18 with an amino acid sequence of G92K/D93F/G95L/A104K mutation into the phospholipid bilayer, the buffer (400mM KCl, 10mM HEPES pH 8.0, 50mM MgCl₂) flowed through the system to remove any excess Mnep-KO nanopores. A DNA construct X2& cX2-80-15 or S1T & SIMC (a final concentration of 1-2 nM) was added to the Mnep-KO nanopore experimental system. After thorough mixing, the buffer (400mM KCl, 10mM HEPES pH 8.0, 50 mM MgCl₂) flowed through the system to remove any excess DNA construct X2& cX2-80-15 or S1T & S1MC. Then, a premix of helicase (EF8813-1, a final concentration of 15nM) and a fuel (ATP, a final concentration of 3mM) was added to the single Mnep-KO nanopore experimental system, and the sequencing of a Mnep-KO porin was monitored at +180 mV.

The same steps were performed in the control group except that the Mnep-KO nanopore was replaced with a wild-type Mnep nanopore (the stick-surface potential model is shown in FIG. 2). Wherein, the stick model of the wild-type Mnep nanopore shows distribution characteristics of amino acids in a narrow region of the pore channel. The critical amino acid residues in the narrow region of the pore channel mainly include serine at position 91, glycine at position 92, aspartic at position 93 and glutamic at position 110 in a loop region. A diameter of narrow regions formed by S91 and E110 was 17.2Å and 18.7Å, respectively. Compared with that of the wild-type Mnep nanopore, the stick-surface potential model of the nanopore including the Mnep monomer variant (the stick- surface potential model is shown in FIG. 1) showed the distribution characteristics of amino acids in the narrow region of the mutant pore channel, in which the distribution of the critical amino acid residues in the narrow region of the mutant pore channel pointed to lysine at position 92 of the amino acid residue in the center of the pore channel, and diameter of pore channel was about 10Å. The side chains of phenylalanine at position 93 and leucine at position 95 swung to the outside of the pore channel, and participated in enhancement of hydrophobic accumulation force in the narrow region of the pore channel. Lysine at position 104 may be closely related to correct assembly of a channel complex.

The specific sequences of X2& cX2-80-15 (a specific structure is shown in FIG. 3) are as follows:
X2:
   TGGTTTTTGTTTGTTTTTAGAATTTTTTTACACTACCACTGCTAGCATTTTTCA (SEQ ID NO: 5)
   TTTCTCACTATCCCGTTCTCATTGGTGCACCATCTTTTTTTGGTT (SEQ ID NO: 6)
   TTTTTGCAGCAGCAT (SEQ ID NO: 7)
cX2-80-15:
   AACCAAAAAAAGATGGTGCACCAATGAGAACGGGATAGTGAGAAA (SEQ ID NO: 8)
   TTTTTTTTTTTTTTTTTTTTTTTTTTTTTTTTTTTTTTTT (SEQ ID NO: 9)

The specific sequences of SIT & S1MC (a specific structure is shown in FIG. 4) are as follows:
S1T:
   TTTTTTTTTTTTTTCCTTCC (SEQ ID NO: 10)
   X (a segment h)
   TTCTTTTCCCGTCCGCTCGT (SEQ ID NO: 12) 
TCGCGCCTGTCTGCTTGTTTTTTTTTTTCTTTTTTTTTTTCTCACTATCGCATTCTC
ATGCAGGTCGGTGGTCGCAGTA (SEQ ID NO: 13)
S1MC:
   ACGAGCGGACGGGAAAAGAA (SEQ ID NO: 14)
   TTTTTTTTTTTTTTTTTTTT (SEQ ID NO: 15)

The sequencing results are shown in FIGS. 7-12, wherein FIG. 7 shows the single channel behavior characteristics of the wild-type Mnep nanopore channel at ±180 mV. In the test system, the wild-type channel has a full open current of about 347 pA at +180 mV, obvious gating and obvious voltage dependence. The full open current was higher at -180 mV, close to -450 pA, and the gating was stronger. Apparently, the wild-type channel cannot meet the requirements of nanoporin and cannot accomplish the sequencing purpose.

FIG. 8 shows the open-pore current and gating characteristics of the Mnep-(G92K/D93F/G95L/A104K) monomer variant at +180 mV, 0 mV and -180 mV. The results showed that the forward gating of the mutant channel disappeared. In the test system, the full open current was about 180 pA at +180 mV, and the gating was stronger at a negative voltage. FIG. 9 shows the signal of nucleic acid passing through the nanopore Mnep-(G92K/D93F/G95L/A104K) at +180 mV.

An example current trajectory when helicase (EF8813-1) controlled a DNA construct X2&cX2-80-15 to translocate through the nanopore including the Mnep-(G92K/D93F/G95L/A104K) monomer variant (see FIGS. 10A, 10B and 10C).

An example current trajectory when helicase (EF8813-1) controlled a DNA construct X2&cX2-80-15 to translocate through the nanopore including the Mnep-(G92K/D93F/G95L/A104K) monomer variant (see FIGS. 11A, 11B, 11C, 11D, 11E and 11F).

An example current trajectory when helicase (EF8813-1) controlled a DNA construct S1T&S1MC to translocate through a mutant Mnep-(G92K/D93F/G95L/A104K) nanopore (see FIGS. 12A, 12B and 12C).

## Claims

1. A *Mycobacterium neoaurum* porin (Mnep) monomer variant, **characterized in that** the Mnep monomer variant comprises an amino acid sequence with amino acid mutations of (1) G92K; (2) D93F; (3) G95L; and (4) A104K, and has at least 95% identity to SEQ ID NO: 1.

2. The Mnep monomer variant according to claim 1, the nucleotide sequence encoding the Mnep monomer variant is shown in SEQ ID NO: 11.

3. A porin comprising at least one Mnep monomer variant, **characterized in that** the Mnep monomer variant comprises an amino acid sequence with amino acid mutations of (1) G92K; (2) D93F; (3) G95L; and (4) A104K, and has at least 95% identity to SEQ ID NO: 1.

4. The porin according to claim3, **characterized in that** the porin comprises 1-20 Mnep monomer variants, wherein the Mnep monomer variants are identical or different; and/or the porin comprises wild-type Mnep monomers.

5. The porin according to claim 4, the Mnep monomer variant and the Mnep monomer variant, the wild-type Mnep monomer and the wild-type Mnep monomer, as well as the Mnep monomer variant and the wild-type Mnep monomer are covalently attached.

6. The porin according to claim 3, **characterized in that** a pore channel diameter of a narrow region of the porin is less than 18.7Å, optionally the pore channel diameter of the narrow region of the porin is less than 17.2Å.

7. A nucleic acid encoding the Mnep monomer variant of any one of claims 1-2 or the porin of any one of claims 3-6.

8. A vector comprising the nucleic acid of claim 7.

9. A mutant bacterium expressing the Mnep monomer variant of any one of claims 1-2 or the porin of any one of claims 3-6.

10. A method for producing a Mnep porin, **characterized in that** the method comprises transforming the mutant bacterium of claim 9 with the vector of claim 8 to induce the mutant bacterium to express the Mnep porin.

11. A method for characterizing a target polynucleotide, **characterized in that** the method comprises the steps of:
(a) contacting the target polynucleotide with the porin of any one of claims 3-6, so that the target polynucleotide sequence passes through the porin; and
(b) obtaining one or more characteristics of an interaction between the nucleotide and the porin when the target polynucleotide passes through the porin to characterize the target polynucleotide.

12. The method according to claim 11, **characterized in that** the step (a) also comprises a step of contacting the target polynucleotide with one or a combination of two or more of nucleic acid binding protein, helicase, polymerase, reverse transcriptase, translocase, exonuclease, telomerase and/or topoisomerase, which makes the translocation speed of the target polynucleotide sequence passes through the porin less than the translocation speed in the absence of the nucleic acid binding protein, helicase, polymerase, reverse transcriptase, translocase, exonuclease, telomerase and/or topoisomerase.

13. The method according to claim 11 or 12, **characterized in that** the method also comprises a step of applying a potential difference across the porin contacted with the target polynucleotide.

## Patentansprüche

1. *Mycobacterium-neoaurum*-Porin(Mnep-)Monomervariante, **dadurch gekennzeichnet, dass** die Mnep-Monomervariante eine Aminosäuresequenz mit Aminosäuremutationen von (1) G92K; (2) D93F; (3) G95L; und (4) A104K umfasst und zumindest 95 % Identität mit SEQ ID NO: 1 aufweist.

2. Mnep-Monomervariante nach Anspruch 1, wobei die Nukleotidsequenz, welche die Mnep-Monomervariante kodiert, gezeigt ist in SEQ ID NO: 11.

3. Porin, umfassend zumindest eine Mnep-Monomervariante, **dadurch gekennzeichnet, dass** die Mnep-Monomervariante eine Aminosäuresequenz mit Aminosäuremutationen von (1) G92K; (2) D93F; (3) G95L; und (4) A104K umfasst und zumindest 95 % Identität mit SEQ ID NO: 1 aufweist.

4. Porin nach Anspruch 3, **dadurch gekennzeichnet, dass** das Porin 1-20 Mnep-Monomervarianten umfasst, wobei die Mnep-Monomervarianten identisch oder unterschiedlich sind; und/oder das Porin Wildtyp-Mnep-Monomere umfasst.

5. Porin nach Anspruch 4, die Mnep-Monomervariante und die Mnep-Monomervariante, das Wildtyp-Mnep-Monomer und das Wildtyp-Mnep-Monomer sowie die Mnep-Monomervariante und das Wildtyp-Mnep-Monomer kovalent gebunden sind.

6. Porin nach Anspruch 3, **dadurch gekennzeichnet, dass** ein Porenkanaldurchmesser einer schmalen Region des Porins weniger als 18,7 Å ist, optional der Porenkanaldurchmesser der schmalen Region des Porins weniger als 17,2 Å ist.

7. Nukleinsäure, welche die Mnep-Monomervariante nach einem der Ansprüche 1-2 oder das Porin nach einem der Ansprüche 3-6 kodiert.

8. Vektor, umfassend die Nukleinsäure nach Anspruch 7.

9. Mutiertes Bakterium, das die Mnep-Monomervariante nach einem der Ansprüche 1-2 oder das Porin nach einem der Ansprüche 3-6 exprimiert.

10. Verfahren zum Produzieren eines Mnep-Porins, **dadurch gekennzeichnet, dass** das Verfahren Transformieren des mutierten Bakteriums nach Anspruch 9 mit dem Vektor nach Anspruch 8 umfasst, um das mutierte Bakterium zu induzieren, das Mnep-Porin zu exprimieren.

11. Verfahren zum Kennzeichnen eines Zielpolynukleotids, **dadurch gekennzeichnet, dass** das Verfahren die folgenden Schritte umfasst:
(a) Kontaktieren des Zielpolynukleotids mit dem Porin nach einem der Ansprüche 3-6, sodass die Zielpolynukleotidsequenz durch das Porin verläuft; und
(b) Erhalten von einer oder mehreren Kennzeichnungen einer Interaktion zwischen dem Nukleotid und dem Porin, wenn das Zielpolynukleotid durch das Porin verläuft, um das Zielpolynukleotid zu kennzeichnen.

12. Verfahren nach Anspruch 11, **dadurch gekennzeichnet, dass** der Schritt (a) auch einen Schritt des Kontaktierens des Zielpolynukleotids mit einem oder einer Kombination von zwei oder mehr von Nukleinsäurebindungsprotein, Helikase, Polymerase, reverser Transkriptase, Translokase, Exonuklease, Telomerase und/oder Topoisomerase umfasst, was die Translokationsgeschwindigkeit der Zielpolynukleotidsequenzläufe durch das Porin weniger als die Translokationsgeschwindigkeit in der Abwesenheit des Nukleinsäurebindungsproteins, der Helikase, der Polymerase, der reversen Transkriptase, der Translokase, der Exonuklease, der Telomerase und/oder der Topoisomerase macht.

13. Verfahren nach Anspruch 11 oder 12, **dadurch gekennzeichnet, dass** das Verfahren auch einen Schritt des Verwendens einer Potentialdifferenz über das Porin, das mit dem Zielpolynukleotid kontaktiert ist, umfasst.

## Revendications

1. Variant de monomère de porine de *Mycobacterium neoaurum* (Mnep), **caractérisé en ce que** le variant de monomère Mnep comprend une séquence d'acides aminés présentant des mutations d'acides aminés de (1) G92K ; (2) D93F ; (3) G95L ; et (4) A104K, et présente au moins 95 % d'identité avec SEQ ID N° : 1.

2. Variant de monomère Mnep selon la revendication 1, la séquence nucléotidique codant pour le variant de monomère Mnep est représentée dans SEQ ID N° : 11.

3. Porine comprenant au moins un variant de monomère de Mnep, **caractérisée en ce que** le variant de monomère Mnep comprend une séquence d'acides aminés présentant des mutations d'acides aminés de (1) G92K ; (2) D93F ; (3) G95L ; et (4) A104K, et présente au moins 95 % d'identité avec SEQ ID N° : 1.

4. Porine selon la revendication 3, **caractérisée en ce que** la porine comprend 1 à 20 variants de monomère Mnep, dans laquelle les variants de monomère Mnep sont identiques ou différents ; et/ou la porine comprend des monomères Mnep de type sauvage.

5. Porine selon la revendication 4, le variant de monomère Mnep et le variant de monomère Mnep, le monomère Mnep de type sauvage et le monomère Mnep de type sauvage, ainsi que le variant de monomère Mnep et le monomère Mnep de type sauvage sont attachés de manière covalente.

6. Porine selon la revendication 3, **caractérisée en ce qu'**un diamètre de canal de pore d'une région étroite de la porine est inférieur à 18,7 Å, éventuellement, le diamètre de canal de pore de la région étroite de la porine est inférieur à 17,2 Å.

7. Acide nucléique codant pour le variant de monomère Mnep de l'une quelconque des revendications 1 à 2 ou la porine de l'une quelconque des revendications 3 à 6.

8. Vecteur comprenant l'acide nucléique de la revendication 7.

9. Bactérie mutante exprimant le variant de monomère Mnep de l'une quelconque des revendications 1 à 2 ou la porine de l'une quelconque des revendications 3 à 6.

10. Procédé de production d'une porine Mnep, **caractérisé en ce que** le procédé comprend la transformation de la bactérie mutante de la revendication 9 avec le vecteur de la revendication 8 pour amener la bactérie mutante à exprimer la porine Mnep.

11. Procédé permettant la caractérisation d'un polynucléotide cible, **caractérisé en ce que** le procédé comprend les étapes de :
(a) mise en contact du polynucléotide cible avec la porine de l'une quelconque des revendications 3 à 6, afin que la séquence de polynucléotide cible passe à travers la porine ; et
(b) obtention d'une ou de plusieurs caractéristiques d'une interaction entre le nucléotide et la porine lorsque le polynucléotide cible passe à travers la porine pour caractériser le polynucléotide cible.

12. Procédé selon la revendication 11, **caractérisé en ce que** l'étape (a) comprend également une étape de mise en contact du polynucléotide cible avec un ou une combinaison de deux éléments ou plus parmi la protéine de liaison à l'acide nucléique, l'hélicase, la polymérase, la transcriptase inverse, la translocase, l'exonucléase, la télomérase et/ou la topoisomérase, ce qui fait que la vitesse de translocation de la séquence polynucléotidique cible passant à travers la porine est inférieure à la vitesse de translocation en l'absence de la protéine de liaison à l'acide nucléique, de l'hélicase, de la polymérase, de la transcriptase inverse, de la translocase, de l'exonucléase, de la télomérase et/ou de la topoisomérase.

13. Procédé selon la revendication 11 ou 12, **caractérisé en ce que** le procédé comprend également une étape d'application d'une différence de potentiel à travers la porine mise en contact avec le polynucléotide cible.
